# EUROPEAN PATENT SPECIFICATION

(11) **EP 3 265 455 B1**
(45) Date of publication and mention of the grant of the patent: **15.07.2020**
(21) Application number: 15883705.4
(22) Date of filing: 04.03.2015
(51) Int. Cl.: C07D 211/28, C07D 211/80, C07D 211/82, C07D 239/42

(54) **PROCESS FOR PREPARING CERITINIB AND INTERMEDIATES THEREOF**
VERFAHREN ZUR HERSTELLUNG VON CERITINIB UND ZWISCHENPRODUKTEN DAVON
PROCÉDÉ DE PRÉPARATION DE CERITINIB ET DE SES INTERMÉDIAIRES

(43) Date of publication of application: 10.01.2018
(73) Proprietor: Novartis AG, 4056 Basel (CH)
(72) Inventor: KAPFERER, Tobias, 4002 Basel (CH); GONG, Baoqing, 4002 Basel (CH); DAVIS, Mark Clinton, 4002 Basel (CH); ZHENG, Xuchun, Changshu Jiangsu 215537 (CN); HAR, Denis, East Hanover, New Jersey 07936 (US)
(74) Representative: Kristl, Jernej
(86) International application number: PCT/CN2015/073647
(87) International publication number: WO 2016/138648

(56) References cited:
- WO-A1-2014/173291
- CN-A- 101 616 895
- CN-A- 103 992 262
- CN-A- 104 109 149
- CN-A- 104 356 050
- CN-A- 104 356 050
- CN-A- 104 356 112
- MARSILJE H T , ET AL: 'Synthesis, Structure?Activity Relationships, and in Vivo Efficacy of the Novel Potent and Selective Anaplastic Lymphoma Kinase (ALK) Inhibitor(LDK378) Currently in Phase 1 and Phase 2 Clinical Trials' JOURNAL OF MEDICINAL CHEMISTRY vol. 56, no. 14, 25 July 2013, pages 5675 - 5690, XP055145299 DOI: 10.1021/JM400402Q

## Description

### Field of the Disclosure

The present disclosure is in the field of organic synthesis and is directed to a method of synthesizing 5-chloro-*N*2-(2-*iso*propoxy-5-methyl-4-(piperidin-4-yl)phenyl)-*N*4-[2-(propane-2-sulfonyl)-phenyl]-pyrimidine-2,4-diamine (ceritinib), a method of preparing an intermediate thereof, and methods for further preparing pharmaceuticals and pharmaceutical compositions from ceritinib or from intermediates.

### Background of the Disclosure

Anaplastic Lymphoma Kinase (ALK) is a member of the insulin receptor superfamily of receptor tyrosine kinases. Chromosomal rearrangements involving ALK has been detected in a variety of human malignancies, such as oncogenesis in hematopoietic and non-hematopoietic tumors, leading to disturbances in the regulation pathway of the cells. Inhibition or suppression of the ALK pathways using an ALK tyrosine kinase inhibitor engenders the cell growth arrest and apoptosis of malignant cells. The study of ALK fusion proteins has also raised the possibility of new therapeutic treatments for patients with ALK positive malignancies.

Ceritinib is an ALK inhibitor with the chemical formula 5-chloro-*N*2-(2-*iso*propoxy-5-methyl-4-(piperidin-4-yl)phenyl)-*N4*-[2-(propane-2-sulfonyl)-phenyl]-pyrimidine-2,4-diamine. A method for preparing it was disclosed in WO2008/073687. WO 2014/173291 discloses deuterated ceritinib and methods for preparing it. CN 104 356 112 A and CN 104 356 050 A disclose methods for preparing ceritinib. CN 104 356 050 A specifically discloses also compound of formula C3, wherein X is -Cl, and a process for preparing it.

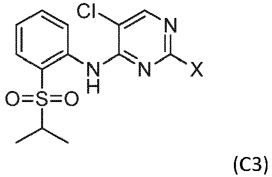

### Summary of the Disclosure

Chemical processes are usually carried out on a small scale in a research/early development phase, and the scale successively increases in late phase development to finally reach the full size production scale. Upon scaling up a process, topics related to process safety are becoming more and more important. Failure to scale up properly may lead to the loss of process control and accidents, such as unexpected exothermic reactions (runaway reactions), health hazards while handling large amount of hazardous and/or toxic chemicals or environmental hazards.

Surprisingly it was found that the process to synthesize ceritinib (5-chloro-*N*2-(2-*iso*propoxy-5-methyl-4-(piperidin-4-yl)phenyl)-*N4*-[2-(propane-2-sulfonyl)-phenyl]-pyrimidine-2,4-diamine) and the intermediates thereof can be prepared with a cost efficient and safer method. Therefore the present disclosure is directed to a new synthesis of ceritinib and its intermediate, using less hazardous chemicals and / or reaction conditions, generating less waste and providing a reproducible process that is easier to handle on a larger scale, a process that is more efficient and generates better quality compounds.

A first aspect of the disclosure is a process for preparing a compound of formula (C2-1), 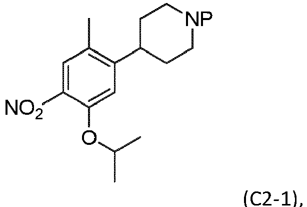 the process comprising the step of reacting a compound of formula (A) 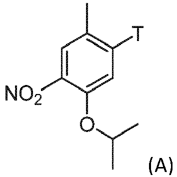 with a compound of formula (B) in a solvent 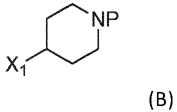 in the presence of at least one catalyst and a co-catalyst or additive, wherein
P is a protecting group;
T and X1 are independently selected from the group consisting of Cl, Br, I, CF₃SO₃, CH₃C₆H₄SO₃, O(t-BuC(O)), MgCl, MgBr, MgI, Sn(Alkyl)₃, Si(Alkyl)₃, Si(OAlkyl)₃, ZnCl, ZnBr, ZnI, ZnAlkyl, B(OH)₂, B(OC(CH₃)₂C(CH₃)₂O), 9-borabicyclo[3.3.1]nonane, bis(1,2-dimethylpropyl)borane, catecholborane, dicyclohexylboronyl, BF₃⁻, B-(N-methyliminodiacetic acid);
the catalyst is Pd(PPh₃)₂Cl₂; and
the co-catalyst or additive is Cul.

Another aspect of the disclosure is a process for preparing ceritinib, or a salt thereof, 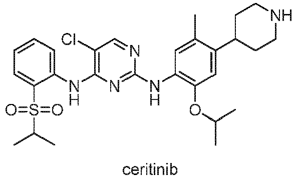 the process comprising the steps of:
i. preparing a compound of formula (C2-1) according to claim 1 or claim 2, 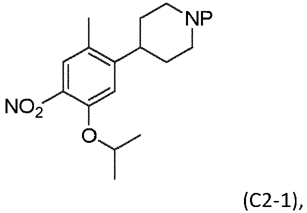
ii. preparing a compound of formula (C2) or a salt thereof from the compound of formula C(2-1), 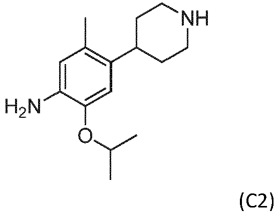
iii. providing a compound (C3), wherein X is selected from halogen (F, Cl, Br, I), alkoxy, aryloxy, alkylthio, sulfinyl, sulfonyl, and 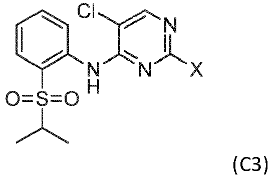
iv. reacting the compound of formula (C2), or a salt thereof, with the compound of formula (C3), to obtain ceritinib, or a salt thereof.

### Detailed Description of the Disclosure

It was observed that increasing the amount of reactants and solvent in order to scale up a reaction to a full size production plant may engender some risks such as loss of process control, unexpected exothermic reactions, accidents and safety issues while handling large amount of hazardous and / or toxic chemicals. Surprisingly, it was found that modifying the process to synthesize ceritinib (5-chloro-*N2*-(2-*iso*propoxy-5-methyl-4-(piperidin-4-yl)phenyl)-*N4*-[2-(propane-2-sulfonyl)-phenyl]-pyrimidine-2,4-diamine) and intermediate provides a scalable method that can safely be handled on a larger scale with reproducible yields, less hazardous / toxic chemicals and produces less waste. In addition, this process produces more efficiently better quality compounds, at a lower cost. A summary of the process is shown below.

### 1.1 Compound of formula (C2-1):

The first aspect of the present disclosure relates to a process for preparing an intermediate compound - a compound of formula (C2-1) 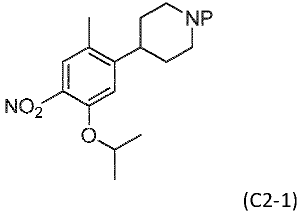 wherein P is a protecting group, or in this case a nitrogen protecting group. The compound of formula (C2-1) can be used for preparing ceritinib.

The term "protecting group" or "nitrogen protecting group" may be present and should protect the functional groups concerned against unwanted secondary reactions, such as acylations, etherifications, esterifications, oxidations, solvolysis and similar reactions. It is a characteristic of protecting groups that they lend themselves readily, *i.e.* without or with very limited undesired secondary reactions, to removal, typically by solvolysis, reduction, photolysis or also by enzyme activity, for example under conditions analogous to physiological conditions, and that they are not present in the end-products. The specialist knows, or can easily establish, which protecting groups are suitable with the reactions mentioned hereinabove and hereinafter. Preferably, if two or more protecting groups are present in one intermediate mentioned, they are chosen so that, if one of the groups needs to be removed, this can be done selectively, *e.g*. using two or more different protecting groups that are cleavable under different conditions, *e.g*. one class by mild hydrolysis, the other by hydrolysis under harder conditions, one class by hydrolysis in the presence of an acid, the other by hydrolysis in the presence of a base, or one class by reductive cleavage (*e.g*. by catalytic hydrogenation), the other by hydrolysis, or the like. Suitable nitrogen protecting groups are conventionally used in peptide chemistry and are described e.g. in the relevant chapters of standard reference works such as J. F. W. McOmie, "Protective Groups in Organic Chemistry", Plenum Press, London and New York 1973; T. W. Greene and P. G. M. Wuts, "Greene's Protective Groups in Organic Synthesis", Fourth Edition, Wiley, New York 2007; in "The Peptides"; Volume 3 (editors: E. Gross and J. Meienhofer), Academic Press, London and New York 1981, and in "Methoden der organischen Chemie" (Methods of Organic Chemistry), Houben Weyl, 4th edition, Volume 15/l, Georg Thieme Verlag, Stuttgart 1974. Preferred nitrogen protecting groups generally comprise: C₁-C₆-alkyl, preferably C₁-C₄-alkyl, more preferably C₁-C₂-alkyl, (*e.g*. acetyl, allyl, *tert*butyl) most preferably C₁-alkyl which is mono-, di- or tri-substituted by trialkylsilyl-C₁-C₇-alkoxy (eg. trimethylsilyethoxy), aryl, preferably phenyl, or an heterocyclic group (*e.g*., benzyl, cumyl, benzhydryl, pyrrolidinyl, trityl, pyrrolidinylmethyl, 1-methyl-1,1-dimethylbenzyl, (phenyl)methylbenzene) wherein the aryl ring or the heterocyclic group is unsubstituted or substituted by one or more, e.g. two or three, residues, e.g. selected from the group consisting of C₁-C₇-alkyl, hydroxy, C₁-Cᵣalkoxy, C₂-C₈-alkanoyl-oxy, halogen, nitro, cyano, and CF₃; aryl-C₁-C₂-alkoxycarbonyl (preferably phenyl-C₁-C₂-alkoxycarbonyl (*eg.* benzyloxycarbonyl (Cbz), benzyloxymethyl (BOM), pivaloyloxymethyl (POM)); C₁-C₁₀-alkenyloxycarbonyl; C₁-C₆alkylcarbonyl (*eg.* acetyl or pivaloyl); C₆-C₁₀-arylcarbonyl; C₁-C₆-alkoxycarbonyl (*eg.* tertbutoxycarbonyl (Boc), methylcarbonyl, trichloroethoxycarbonyl (Troc), pivaloyl (Piv), allyloxycarbonyl); C₆-C₁₀-arylC₁-C₆-alkoxycarbonyl (*e.g.* 9-fluorenylmethyloxycarbonyl (Fmoc)); allyl or cinnamyl; sulfonyl or sulfenyl; succinimidyl group, silyl groups (*e.g*. triarylsilyl, trialkylsilyl, triethylsilyl (TES), trimethylsilylethoxymethyl (SEM), trimethylsilyl (TMS), tri*iso*propylsilyl or *tert*butyldimethylsilyl).

According to the disclosure the preferred protecting group (P) can be selected from the group consisting of tert-butyloxycarbonyl (Boc), benzyloxycarbonyl, methyloxycarbonyl or benzyl.

A compound of formula (C2-1) can be prepared by a process which comprises reacting a compound of formula (A) with a compound of formula (B) in a solvent in the presence of at least one catalyst, optionally a co-catalyst or additive, as defined in Scheme 2. The reaction is normally heated. Suitable solvents used for the reaction are, for example, tetrahydrofuran (THF), 2-methyl-tetrahydrofuran, 1,4-dioxane, diethyl ether, toluene, dimethylformamide (DMF), dimethylacetamide (DMA), dimethylsulfoxide (DMSO), dimethoxyethane (DME), dichloromethane, *N*-methyl-2-pyrrolidone (NMP), 1-butyl-2-pyrrolidone (NBP), acetonitrile, acetone, ethylacetate, *iso*propylacetate, *tert*butylacetate, pentane, hexane, heptane, anisole, pyridine, triethylamine, dimethycarbonate, water, methanol, ethanol, *n*-propanol, 2-propanol, *n-*butanol, 2-butanol, *tert*-butanol, or mixtures thereof.

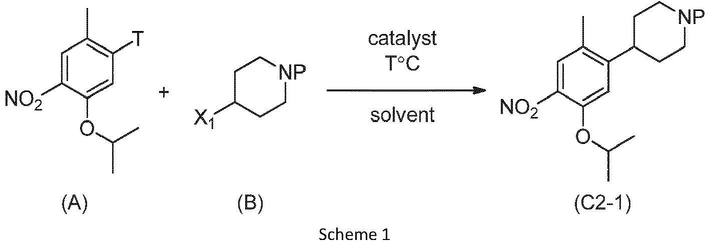

Although selection of a suitable protecting group (P) is vast, the preferred protecting group (P) is *tert-*butyloxycarbonyl (Boc), benzyloxycarbonyl, methyloxycarbonyl, ethyloxycarbonyl, allyloxycarbonyl, phenyloxycarbonyl, formyl, acetyl or benzyl. The most preferred protecting group in this process is is *tert-*butyloxycarbonyl (Boc), benzyloxycarbonyl, methyloxycarbonyl or benzyl.

In the reaction Scheme 2, T and X₁ can for example be independently selected from the group consisting of Cl, Br, I, OTf, OTs, OPiv or T can be a metal species M selected from ZnCl, ZnBr, ZnI, Zn(Alkyl), B(OH)₂, B(OC(CH₃)₂C(CH₃)₂O), 9-BBN, B(Sia)₂, B(Cat), B(Cy)₂, BF₃⁻ or B(MIDA). Generally, T and X₁ can for example independently be Cl, Br, I, OTf, OTs, OPiv, MgCl, MgBr, MgI, Sn(Alkyl)₃, Si(Alkyl)₃, Si(OAlkyl)₃, ZnCl, ZnBr, ZnI, Zn(Alkyl), B(OH)₂, B(OC(CH₃)₂C(CH₃)₂O), 9-BBN, B(Sia)₂, B(Cat), B(Cy)₂, BF3⁻, B(MIDA).

The term "Alkyl" herein refers to a radical or part of a radical that is a straight or branched (one or, if desired and possible, more times) carbon chain. It can be C₁-C₈-alkyl. The term "C₁-C₈-" defines a moiety with up to and including maximally 8 carbon atoms, said moiety being branched (one or more times) or straight-chained and bound *via* a terminal or a non-terminal carbon. C₁-C₈-alkyl, for example, is *n*-pentyl, *n*-hexyl or *n*-heptyl, *n*-octyl, methyl, ethyl, *n*-propyl, *iso*propyl, *n*-butyl, *iso*butyl, *sec-*butyl, *tert*-butyl, in particular methyl, ethyl, *n*-propyl, *iso*propyl, *n*-butyl, *iso*butyl, *sec*-butyl, *tert*-butyl.

The catalyst used to perform the reaction outlined in Scheme 2 is Pd(PPh₃)₂Cl₂.

The co-catalyst or additive is CuI.

The catalyst can be generally present in an amount up to 10.0 mol%. Typically, the catalyst may be present in an amount below 5.0 mol%. The catalyst can be further present in a range from about 0.005 mol% to about 5.0 mol%, about 0.01 mol% to about 1.0 mol%, or from about 0.05 mol% to about 0.5 mol%, based on the starting compound of formula (A). Typically, the catalyst may be present in an amount of about 0.1 mol%.

The reaction described in Scheme 2 is performed particularly well when T is Br, X₁ is ZnI and P is *tert-*butyloxycarbonyl (Boc). The preferred solvent for the reaction is tetrahydrofuran (THF), the catalyst is Pd(PPh₃)₂Cl₂ and the co-catalyst or additive is CuI. Particularly the reaction performs well at a temperature over 25°C, preferably about 50°C.

The term "co-catalyst or additive" as used herein refers to a chemical agent that enhances the rate of a chemical reaction by lowering the activation energy.

### 1.2 Compound of formula (C2):

A compound of formula (C2), or a salt thereof, can be prepared according to the process summarized in Scheme 4. The process involves reducing and deprotecting a compound of formula (C2-1) as disclosed herein to prepare a compound of formula (C2), or a salt thereof.

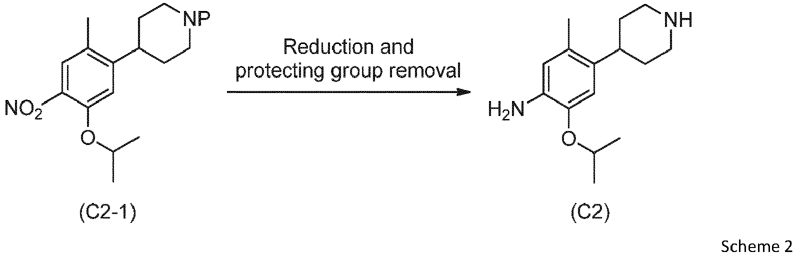

The reduction of compound (C2-1) that possesses a protecting group P as described for Scheme 2, can be performed with hydrogen in the presence of a catalyst. The protecting group P refers to a nitrogen protecting group selected from the above mentioned list (for example *tert*-butyloxycarbonyl (Boc), benzyloxycarbonyl, methyloxycarbonyl, ethyloxycarbonyl, allyloxycarbonyl, phenyloxycarbonyl, formyl, acetyl or benzyl).

The catalyst used to perform the reduction of compound of formula (C2-1) is any catalyst that a skilled person would select from a general textbook. The catalyst can be selected from the group consisting of Raney nickel, Pt/C, Rh/C, Pd/Al₂O₃, Pd/CaCO₃, RhCl(PPh₃)₃, Lindlar catalyst, PtO₂, Pd/C, [Rh(cod)(PPh₃)₂]⁺, [lr(cod)(PCy₃)(Py)⁺, Pd(OH)₂, Pd(OAc)₂, Pd₂(dba)₃, Zn, Fe, Sm, NiCl₂, Ni(OAc)₂, CoCl₂, ZrCl₄, TiCl₃. The catalyst can be present in a range from about 0.005 mol% to about 20.0 mol%. Typically, the catalyst can be present in an amount below 10.0 mol% (to about 0.005 mol%).

The term "catalyst" as used herein refers to a catalytic amount of a chemical agent that enhances the rate of a chemical reaction by lowering the activation energy for the chemical reaction. The catalyst can be a heterogeneous catalyst or a homogenous catalyst.

The term "heterogeneous catalyst" refers to a catalyst supported on a carrier, typically although not necessarily a substrate comprised of an inorganic material, for example, a porous material such as carbon, silicon and / or aluminum oxide.

The term "homogeneous catalyst" refers to a catalyst that is not supported on a carrier.

The terms "hydrogen" or "hydrogenation" used to describe a chemical reaction refer to the action of reducing another compound in the presence of hydrogen. The source of hydrogen can be selected from gaseous hydrogen (H₂), hydrogen donors (transfer hydrogenation, e.g. formic acid or salts thereof), hydride reagent (BH₃, B₂H₆. NaBH₄) or the like.

The reduction may be performed in a solvent such as an alcohol based solution. The alcohol based solution can comprise or consist of C₁ to C₁₀ alcohols (*e.g*. methanol, ethanol, propanol, *iso*propanol and butanol) or mixtures thereof.

The reaction of reduction is best carried out at elevated pressure, preferably between 1 bar and 10 bar, particularly at 4 bar. Reduction of the compound of formula (C2-1), or a salt thereof, is best done by stirring the reaction mixture for 5 hours at room temperature, with 10 mol% of Pd/C and hydrogen in the presence of ethanol. The reduction is best performed with the protecting group P being selected from *tert*-butyloxycarbonyl (Boc) or methyloxycarbonyl.

The term "room temperature" or "ambient temperature" as used herein, unless specified otherwise, means a temperature from 15 to 30 °C, such as from 20 to 30 °C, particularly such as from 20 to 25 °C.

In addition to reducing the compound (C2-1) the protecting group gets cleaved off. The removal of the protecting group can be carried out under standard reaction conditions known in the art. Unless otherwise specified, the protecting group can be removed in the absence or, customarily, in the presence of acids or bases, preferably acids or bases that cause removal of the protecting group but at the same time do not cause chemical degradation of the compounds and intermediates. The removal of the protecting group can also be carried out under reductive conditions, e.g. during the first nitro reduction step of (C2-1). Preferably, the protecting group is removed with an acid. Particularly suitable acids for the removal of the protecting group P are HF.pyridine, HF.triethylamine ammonium fluoride, hexafluoro*iso*propanol, acetic acid, trifluoroacetic acid, hydrochloric acid, sulfuric acid, or a combination thereof. Preferably the acid is trifluoroacetic acid or hydrochloric acid. The reaction consisting of removing the protecting group can take place in a solvent that facilitates the removal of the protecting group. As an example, the protecting group can be removed in a solvent selected from the group consisting of dichloromethane, ethyl acetate, 1,4-dioxane, diethyl ether, tetrahydrofuran (THF), methanol or acetonitrile. The protecting group is removed by stirring the reaction mixture for at least 8 hours, preferably for 16 hours at a temperature between -78°C and 70°C, preferably between 0°C and 70°C. The deprotection reaction for the *tert*-butyloxycarbonyl (Boc) protecting group is performed best with trifluoroacetic acid in dichloromethane, optionally at ambient temperature. The deprotection reaction for the methyloxycarbonyl protecting group is performed best with hydrochloric acid, optionally at 60°C.

Reduction and protecting group removal can be performed simultaneously.

The term "alkoxy", being a radical or part of a radical, refers to alkyl-O-, wherein the term alkyl is as defined herein, and includes, for example, C₁-C₂₀-alkoxy (-O-C₁-C₂₀-alkyl), preferably C₁-C₇-alkoxy (-O-C₁-C₇-alkyl). In particular, alkoxy includes, for example, methoxy (OMe), ethoxy (OEt), *n*-propyloxy, *iso*propyloxy, *n*-butyloxy, *iso*butyloxy, *sec*-butyloxy, *tert*-butyloxy (O*t*Bu), pentyloxy, hexyloxy and heptyloxy radicals. The preferred alkoxy substitutents are methoxy (OMe), ethoxy (OEt) or *tert*-butyloxy (O*t*Bu).

The term "aryl", being a radical or part of a radical, refers to an aromatic hydrocarbon group, for example, C₆-C₁₀-aryl, and is preferably a mono- or polycyclic, especially monocyclic, bicyclic or tricyclic aryl moiety with 6 to 14 carbon atoms, for example 6 to 10 carbon atoms. Preferably aryl denotes phenyl, benzyl, indenyl, indanyl or naphthyl. The term "Aryloxy" refers to an Aryl-O-, wherein aryl is as defined above. In particular, the preferred aryloxy herein is phenoxy (OPh). The term "aryllthio" refers to Aryl-S-, wherein aryl is as defined above. The preferred arylthio is benzylthio (SCH₂Ph).

The term "alkylthio" refers to alkyl-S-, wherein alkyl is as defined herein. The alkyl group for example comprises 1 to 8 carbon atoms. In particular, alkylthio includes, for example, methylthio (SMe), ethylthio (SEt), phenylthio (PhS) and pentylthio. The preferred alkylthio substituents herein are methylthio (SMe) and ethylthio (SEt).

The term "sulfinyl" corresponds to a -S-O-alkyl group that includes C₁-C₈alkyl linear or branched. In particular, sulfinyl includes, for example, methylsulfinyl (SOMe), ethylsulfinyl (SOEt), phenylsulfinyl (SOPh) and benzylsulfinyl (SOCH₂Ph).

The term "sulfonyl" refers to the divalent -S(O)₂- group. In particular, sulfonyl includes, for example methylsulfonyl, ethylsulfonyl, phenylsulfonyl and benzylsulfonyl.

### 1.3 Compound of formula (C3):

From the compound of formula (C3-1), a compound of formula (C3) can be prepared. As shown in Scheme 8, the compound (C3) can be prepared by oxidizing the obtained compound of formula (C3-1), wherein X is selected from halogen (F, Cl, Br, I), alkoxy (preferably OMe, OEt, OtBu) and aryloxy (preferably OPh), sulfinyl (preferably SOMe, SOEt, SOCH₂Ph), sulfonyl (preferably SO₂Me, SO₂Et, SO₂CH₂Ph), most preferably X is Cl.

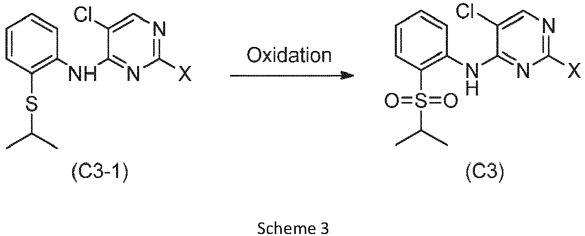

The oxidation can be performed in water or an organic solvent. The solvent can be selected from 1,4-dioxane, tetrahydrofuran (THF), 2-methyl tetrahydrofuran, diethyl ether, toluene, dimethylformamide (DMF), dimethylacetamide (DMA), dimethylsulfoxide (DMSO), dimethoxyethane (DME), dichloromethane, *N*-methyl-2-pyrrolidone (NMP), 1-butyl-2-pyrrolidone (NBP), acetonitrile, acetone, dimethylcarbonate, ethyl acetate, *iso*propylacetate, *tert*butylacetate, pentane, hexane, heptane, anisole, pyridine, triethylamine, acetic acid, water, methanol, ethanol, *n*-propanol, 2-propanol, *n*-butanol, 2-butanol and tert-butanol, or mixtures thereof.

The oxidative reagent can be selected, among others, from the group consisting of KMnO₄, MnO₂, NalO₅, NaClO, KHSO₅ (Oxone), NaBO₃, CH₃CO₃H, H₂O₂, Na₂WO₄, O₂, O₃, tetrapropylammonium perruthenate (TPAP), 3,3-dimethyldioxirane, 3-chloroperoxybenzoic acid (*m*CPBA) and tertbutylhydroperoxide (TBHP), or mixtures thereof, optionally with a catalyst.

Preparing the compound of formula (C3) from the compound of formula (C3-1) can include stirring the reaction mixture for 4 to 40 hours, preferably for about 16 hours at a temperature between 10 and 60 °C, preferably between 20 and 40 °C, particularly at about 30 °C. The oxidation of the compound of formula (C3-1) can be carried out in high yield in ethyl acetate, in the presence of CH₃CO₃H as a solution in CH₃CO₂H at 30 °C or the process can be carried out in high yield in methanol, in the presence of H₂O₂ and Na₂WO₄ at the temperature between 20 - 70 °C.

The compound of formula (C3) can alternatively be prepared by following the steps of (i) reacting a compound of formula (H) with a compound of formula (I) to obtain an intermediate; (ii) reducing the intermediate to form compound (J); and (iii) reacting the compound (J) with a compound of formula (G) in the presence of a base, as described in Scheme 9, *vide infra.*

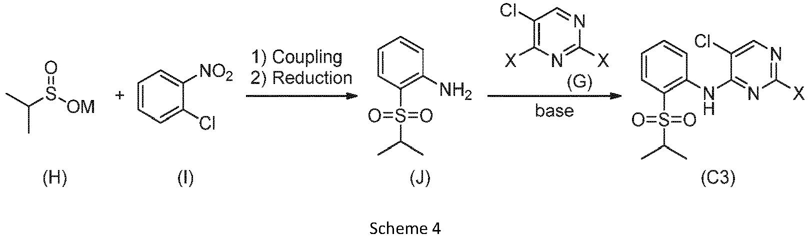

X can denote, as above, F, Cl, Br, I, Alkoxy (preferably OMe, OEt, OtBu), Aryloxy (preferably OPh), alkylthio (preferably SMe, SEt), arylthio (preferably SCH₂Ph), sulfinyl (preferably SOMe, SOEt, SOCH₂Ph), sulfonyl (preferably SO₂Me, SO₂CH₂Ph). Most preferably X is Cl. M can be selected from Li, Na, K, 0.5 Zn, 0.5 Ca, preferably M is Na.

Compound of formula (H) and the compound of formula (I) react in a solvent while being stirred at a temperature between the room temperature and reflux. In specific embodiment the reaction is conducted in a solvent at a temperature between 82-86 °C.

The solvent used for the reaction can be for example dimethylsulfoxide (DMSO), 1,4-dioxane, tetrahydrofuran (THF), 2-methyl tetrahydrofuran, diethyl ether, toluene, dimethylformamide (DMF), dimethylacetamide (DMA), dimethoxyethane (DME), dichloromethane, *N*-methyl-2-pyrrolidone (NMP), 1-butyl-2-pyrrolidone (NBP), acetonitrile, acetone, dimethylcarbonate, ethyl acetate, *iso*propylacetate, tertbutylacetate, pentane, hexane, heptane, anisole, pyridine, triethylamine, acetic acid, water, methanol, ethanol, *n*-propanol, 2-propanol, *n*-butanol, 2-butanol, *tert*-butanol, or mixtures thereof. Preferably DMSO is selected.

The reductive step to obtain a compound of formula (J) can include use of a catalyst and hydrogen in a solvent. The catalyst used to perform the reduction is any catalyst that a skilled person would know to select from a general textbook. The catalyst can be for example Raney nickel, Pt/C, Rh/C, Pd/Al₂O₃, Pd/CaCO₃, RhCl(PPh₃)₃, Lindlar catalyst, PtO₂, Pd/C, [Rh(cod)(PPh₃)₂]⁺, [Ir(cod)(PCy₃)(Py)]⁺, Pd(OH)₂, Pd/Al, Pt/Al, Pt/SiAl, Pd/ZrO₂, or mixtures thereof.

The catalyst added to the reaction mixture can be present in a range from about 0.005 mol% to about 50.0 %w/w (dry) based on the starting compound of formula (I). Typically, the catalyst may be present in an amount below 20.0 % w/w (dry).

The reduction reaction can be stirred for several hours, normally at a temperature up to 60°C, preferably about 40°C.

The reaction of reduction is best carried out at elevated pressure, for example pressure between 1 and 15 bar, preferably between 2 and 6 bar.

The reduction may be performed in a solvent such as an alcohol based solution. The alcohol based solution can be a C₁ to C₁₀ alcohols (*e.g*. methanol, ethanol, propanol, *iso*propanol and butanol) or mixtures thereof may be used as the reaction medium. Preferably the solvent is ethanol.

The compound of formula (C3) is obtained by reacting the intermediate of formula (J) with a compound of formula (G) in the presence of a base and in the absence of a solvent. Performing the reaction without a solvent is a really attractive alternative as it is more efficient, engender less waste and reduces the overall cost of the synthesis.

The base used to perform the reaction is any base that a skilled person would select based on a general textbook. The base can be for example selected from the group consisting of Na₂CO₃, K₂CO₃, Cs₂CO₃, NaHCO₃, KHCO₃, triethylamine, DIPEA, Na₃PO₄, K₃PO₄, DBU and NaH. Best conditions are achieved when DBU or DIPEA are used, particularly DBU. The mild base defined herein allows to achieve an efficient process, is easy and safe to handle, and potentially prevents side reactions and thus allows recovery of compound of formula (C3) in high yield and purity.

The reaction between intermediate (J) and compound of formula (G) is best performed at a temperature between 40°C and reflux, particularly at 80°C.

### 1.4 Ceritinib:

Aforementioned processes can be extended to prepare ceritinib or a salt thereof. Depending on the starting materials and the selected route, a skilled person would know how to combine them to produce building blocks to eventually form ceritinib. Certain variants or alternative processes are described herein below. For example, as shown in Scheme 10, ceritinib, or a salt thereof, can be prepared in a process, the process comprising the steps of:
i. preparing a compound of formula (C2-1) as described in 1.1
ii. preparing a compound of formula (C2), or a salt thereof,
iii. providing a compound (C3), and
iv. reacting a compound of formula (C2) with a compound of formula (C3), to obtain ceritinib, or a salt thereof.

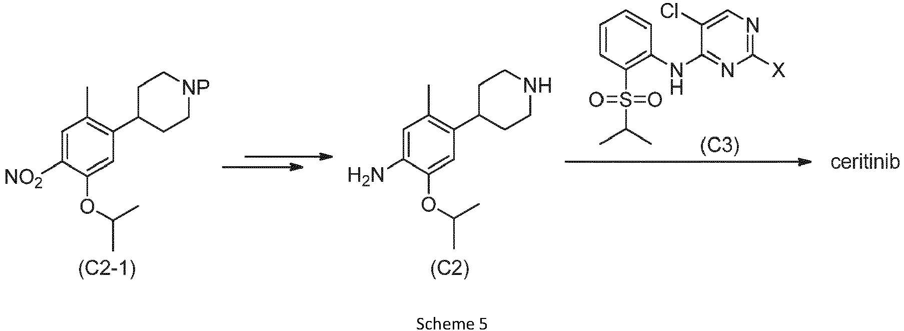

The compound of formula (C2), or a salt thereof, and the compound of formula (C3) can be coupled in a solvent in the presence of a base, wherein X can be halogen (Br, Cl, I), alkoxy (preferably OMe, OEt, OtBu) and aryloxy (preferably OPh), sulfinyl (preferably SOMe, SOEt, SOCH₂Ph) or sulfonyl (preferably SO₂Me, SO₂Et, SO₂CH₂Ph); most preferably X is Cl.

The base for the reaction can be selected from a group of mild bases such as Na₂CO₃, K₂CO₃, Cs₂CO₃, NaHCO₃, KHCO₃, triethylamine, DIPEA, DBU, Na₃PO₄ or K₃PO₄. Alternatively, the base can be omitted.

The reaction can be controlled in vast variety of solvents, for example 1,4-dioxane, tetrahydrofuran (THF), 2-methyl tetrahydrofuran, diethyl ether, toluene, *N*-methyl-2-pyrrolidone (NMP), 1-butyl-2-pyrrolidone (NBP), acetonitrile, acetone, dimethylcarbonate, ethylacetate, *iso*propylacetate, *tert*butylacetate, water, methanol, ethanol, *n*-propanol, *iso*propanol, *n*-butanol, 2-butanol, *tert*-butanol and toluene, or mixtures thereof. Preferably tetrahydrofuran (THF), 2-methyl tetrahydrofuran, water, methanol, ethanol, *n-*propanol, *iso*propanol, *n*-butanol, 2-butanol are used, most preferably *iso*propanol.

The process of reacting the compound of formula (C2), or a salt thereof, to yield the compound of formula (C3) is done by stirring the reaction mixture for 6 to 41 hours, preferably for about 16 hours at a temperature between 40°C and reflux temperature, preferably at reflux.

The process of reacting a compound of formula (C2) and a compound of formula (C3) is best carried out in *iso*propanol, at reflux, without a base, hence preventing side reactions and allowing the recovery of ceritinib, or a salt thereof, in high yield and purity.

When salts are referred to herein, it is meant especially pharmaceutically acceptable salts or other generally acceptable salts, unless they would be excluded for chemical reasons, which the skilled person will readily understand. Salts can be formed with final products or intermediates where salt forming groups, such as basic or acidic groups, are present that can exist in dissociated form at least partially, e.g. in a pH range from 4 to 10 in aqueous solutions, or can be isolated especially in solid, especially crystalline, form.

Such salts are formed, for example, as acid addition salts, preferably with organic or inorganic acids, from compounds or any of the intermediates mentioned herein with a basic nitrogen atom (e.g. imino or amino), especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, lactic acid, fumaric acid, succinic acid, citric acid, amino acids, such as glutamic acid or aspartic acid, maleic acid, hydroxymaleic acid, methylmaleic acid, benzoic acid, methane- or ethane-sulfonic acid, ethane-1,2-disulfonic acid, benzenesulfonic acid, 2-naphthalenesulfonic acid, 1,5-naphthalene-disulfonic acid, N-cyclohexylsulfamic acid, N-methyl-, N-ethyl- or N-propyl-sulfamic acid, or other organic protonic acids, such as ascorbic acid.

Ceritinib prepared as described above may optionally be further purified by recrystallisation from a suitable solvent and may optionally be milled or sieved in order to obtain the final pharmaceutically active ingredient.

Once the pharmaceutically active ingredient ceritinib is obtained (for example as described in Section 1.8 above) it can be mixed with a pharmaceutically acceptable excipient. This can be achieved by mixing, granulating, compacting and the like. This way, a pharmaceutical composition can be prepared and used for the preparation of final dosage forms, such as tablets or capsules.

As used in this specification and the appended claims, the singular forms "a", "an", and "the" include plural referents unless the context clearly indicates otherwise.

Similarly, "comprise", "comprises", "comprising", "include", "includes" and "including" are interchangeable and not intended to be limiting.

### Abbreviations

- δ: Chemical shift
- ¹³C-NMR: Carbon nuclear magnetic resonance
- ¹H-NMR: Proton nuclear magnetic resonance
- Ac: Acetyl
- Acac: Acetylacetone
- AcOH: Acetic acid
- ALK: Anaplastic Lymphoma Kinase
- Amphos: Bis(di-*tert*-butyl(4-dimethylaminophenyl)phosphine)
- B(cat): Catecholborane
- B(Sia)₂: Bis(1,2-dimethylpropyl)borane
- BBN: Borabicyclo[3.3.1]nonane
- BINAP: (1,1'-Binaphthalene-2,2'-diyl)bis(diphenylphosphine)
- Boc: *Tert*-butyloxycarbonyl
- BOM: Benzyloxymethyl
- br: broad
- br m: Broad multiplet
- br s: Broad singlet
- Bu: Butyl
- CaCO₃: Calcium carbonate
- cat.: Catalytic amount
- Cbz: Benzyloxycarbonyl
- CDCl₃: Deuterated chloroform
- CH₃CO₃H: Peracetic acid
- cod: Cyclo-1,5-octadiene
- cy: Cyclohexyl
- d: Doublet
- dba: dibenzylideneacetone
- DBU: 1,8-Diazabicycloundec-7-ene
- dcpp: 1,3-Bis(dicyclohexylphosphanyl)propane
- DIPEA: *N*,*N*-Di*iso*propylethylamine or Hünig's base
- DMA: dimethylacetamide
- DME: dimethoxyethane
- DMF: Dimethylformamide
- DMSO: dimethylsulfoxide
- DMSO-*d*₆: Dimethylsulfoxide deuterated
- DPE-phos: Bis(2-diphenylphosphinophenyl)ether
- DPE-phos: Bis(2-diphenylphosphinophenyl)ether
- Dppb: 1,4-Bis(diphenylphosphino)butane
- Dppe: 1,2-Bis(diphenylphosphino)ethane
- Dppf: 1,1'-bis(diphenylphosphanyl)ferrocene
- Dppp: 1,3-Bis(diphenylphosphanyl)propane
- Eq.: equivalent
- Et: Ethyl
- Fmoc: 9-Fluorenylmethyloxycarbonyl
- g: Gram(s)
- h: Hour(s)
- H₂O₂: Hydrogen peroxide
- HF: Hydrogen fluoride
- Hz: Hertz
- *J*: Coupling constant
- K₂CO₃: Potassium carbonate
- KH: Potassium hydride
- KOAc: Potassium acetate
- LCMS: liquid chromatography-mass spectrometry
- m: multiplet
- M: molarity/molar
- *m*CPBA: 3-chloroperoxybenzoic acid
- Me: Methyl
- mg: Milligram(s)
- MIDA: *N*-Methyliminodiacetic acid
- min: Minutes
- ml: milliliter
- mol: Mole(s)
- Ms: Mesyl
- Na₂WO₄: Sodium tungstate dihydrate
- NaH: Sodium hydride
- NBP: 1-butyl-2-pyrrolidone
- NMP: *N*-methyl-2-pyrrolidone
- Pd/Al: Palladium on aluminum
- Pd/C: Palladium on Carbon
- Pd-PEPPSI-Br: [1,3-Bis(2,6-di*iso*propylphenyl)imidazole-2-ydilene](3-chloropyridyl)palladium(II) dichloride
- PEPPSI: Pyridine enhanced precatalyst preparation stabilization and initiation
- Ph: Phenyl
- phos: Phosphine
- piv: Pivaloyl
- POM: pivaloyloxymethyl
- ppm: parts per million
- Pt/C: Platinium on carbon
- Rh/C: Rhodium on carbon
- s: singlet
- SEM: Trimethylsilylethoxymethyl
- Sept.: septuplet
- T°C: Temperature in celsius
- TBHP: *tert*butylhydroperoxide
- TBME: Methyl *tert*butylether
- *t*Bu: *tert*butyl
- TES: Triethylsilyl
- Tf: Triflate
- THF: tetrahydrofuran
- TPAP: tetrapropylammonium perruthenate
- troc: Trichloroethoxycarbonyl
- Ts: Tosyl
- w/w: Percentage by weight

### Examples

The Following examples are illustrative of the present disclosure.

### Synthesis of 2-isopropoxy-5-methyl-4-(piperidin-4-yl)aniline dihydrochloride (C2, di-HCl salt) according to the following sequence:

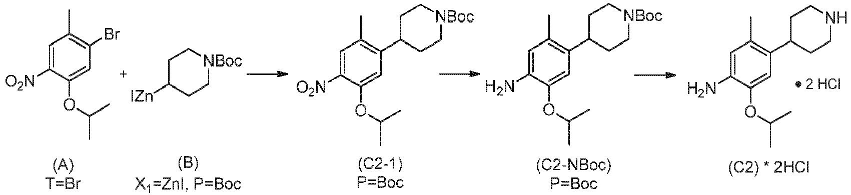

### Tert-butyl 4-(5-isopropoxy-2-methyl-4-nitrophenyl)piperidine-1-carboxylate (C2-1, P=Boc)

To a mixture of Pd(PPh₃)₂Cl₂ (69 mg, 0.099 mmol, 1.5 mol%), CuI (75 mg, 0.39 mmol, 6 mol %), 1-bromo-5-*iso*propoxy-2-methyl-4-nitrobenzene (1.80 g, 6.57 mmol) in tetrahydrofuran (19 ml), a solution of (1-(*tert*-butoxycarbonyl)piperidin-4-yl)zinc(II) iodide in tetrahydrofuran (4.95 g of a 0.9 M solution, 13.1 mmol, 2.0 eq.; prepared according to literature procedure) was added at 50 °C and the reaction mixture stirred for 21 hours at this temperature. After cooling to room temperature, saturated aqueous solution of NH₄Cl (50 ml) was added. The aqueous phase was extracted with ethyl acetate (3 x 70 ml), the combined organic phases washed with brine (80 ml) and dried over Na₂SO₄. The volatiles were removed under vacuum and the crude material was purified by silica gel chromatography (ethyl acetate/heptane) to yield tert-butyl 4-(5-*iso*propoxy-2-methyl-4-nitrophenyl)piperidine-1-carboxylate (1.96 g, 79% yield) as a brownish oil. ¹H-NMR (400 MHz, CDCl₃): δ = 1.30 (d, *J* = 6.1 Hz, 6H), 1.42 (s, 9H), 1.47-1.54 (m, 2H), 1.67-1.70 (m, 2H), 2.24 (s, 3H), 2.72-2.82 (m, 3H), 4.05 (br. m, 2H), 4.53 (sept, *J* = 6.1 Hz, 1H), 6.79 (s, 1H), 7.55 (m, 1H) ppm.

### Tert-butyl 4-(4-amino-5-isopropoxy-2-methylphenyl)piperidine-1-carboxylate (C2-NBoc, P=Boc)

To a solution of *tert*-butyl 4-(5-*iso*propoxy-2-methyl-4-nitrophenyl)piperidine-1-carboxylate (1.96 g, 5.18 mmol) in ethanol (180 ml), Pd/C (10%, 0.4 g) was added and the reaction mixture was stirred for 5 hours at room temperature under a hydrogen atmosphere (4 bar). The hydrogen atmosphere was released, the reaction vessel purged with argon, and the mixture filtered through Celite®. The volatiles were removed under vacuum to obtain tert-butyl 4-(4-amino-5-*iso*propoxy-2-methylphenyl)piperidine-1-carboxylate (1.67 g, 92% yield) as a yellow oil, which was used in the next step without further purification.

### 2-Isopropoxy-5-methyl-4-(piperidin-4-yl)aniline dihydrochloride (C2, di-HCl salt)

*Tert*-butyl 4-(4-amino-5-*iso*propoxy-2-methylphenyl)piperidine-1-carboxylate (1.67 g) was dissolved in dichloromethane (10 ml) and the resulting solution treated with trifluoroacetic acid (2 ml). After 16 hours at room temperature, water (50 ml) was added. The phases were separated and the aqueous phase washed with dichloromethane (30 ml). The aqueous phase was neutralized with aqueous NaOH (1 M solution) and extracted with toluene (3 x 50 ml). To the combined toluene phases, HCl (3.8 ml of a 5 M solution in *iso*propanol) was added. After evaporation, 2-*Iso*propoxy-5-methyl-4-(piperidin-4-yl)aniline dihydrochloride was obtained (1.35 g, 88% yield). ¹H-NMR (400 MHz, D₂O): δ = 1.24 (d, *J* = 6.1 Hz, 6H), 1.76-1.96 (m, 4H), 2.22 (s, 3H), 3.04-3.14 (m, 3H), 3.45-3.49 (m, 2H), 4.71 (sept, *J* = 6.1 Hz, 1H; overlapped with the solvent signal), 6.95 (s, 1H), 7.13 (s, 1H) ppm. ¹³C-NMR (100 MHz, D₂O): δ = 17.4, 20.9, 28.6, 35.4, 44.4, 72.3, 112.2, 117.6, 125.4, 128.9, 144.6, 148.8 ppm.

### Synthesis of 2,5-dichloro-N-(2-(isopropylsulfonyl)phenyl)pyrimidin-4-amine (C3, X=Cl), according to the following sequence:

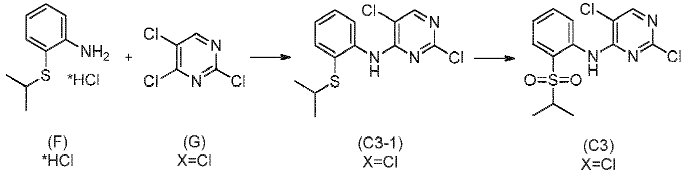

### 2,5-Dichloro-N-(2-(isopropylthio)phenyl)pyrimidin-4-amine (C3-1, X=Cl)

*N*,*N*-Di*iso*propylethylamine (31.0 g, 0.24 mol, 2.3 eq.) was added to a mixture of 2-(*iso*propylthio)aniline hydrochloride (21.3 g, 0.10 mol), 2,4,5-trichloropyrimidine (19.0 g, 0.10 mol, 1.0 eq.), in toluene (166 g) and *n*-butanol (17 g). The mixture was refluxed for about 22 hours. After cooling the reaction mixture to 25 °C, water (70 g) was added, the phases were separated, and the organic phase was washed with water (70 g). The organic phase was concentrated under vacuum, followed by addition of ethanol (34 g). The mixture was refluxed and slowly cooled to 0-5 °C, filtered and dried under vacuum to yield 2,5-dichloro-*N*-(2-(*iso*propylthio)phenyl)pyrimidin-4-amine (26.4 g, 81% yield). ¹H-NMR (400 MHz, CDCl₃): δ = 1.30 (d, *J* = 7.0 Hz, 6H), 3.18 (sept, *J* = 6.7 Hz, 1H), 7.11-7.14 (m, 1H), 7.46-7.50 (m, 1H), 7.60-7.62 (m, 1H), 8.25 (s, 1H), 8.67-8.68 (m, 1H), 9.30 (br. s, 1H) ppm. ¹³C-NMR (100 MHz, CDCl₃): δ = 23.3, 40.7, 114.9, 119.8, 122.9, 124.0, 130.4, 137.1, 139.8, 154.6, 156.0, 158.1 ppm.

### 2,5-Dichloro-N-(2-(isopropylsulfonyl)phenyl)pyrimidin-4-amine (C3)

To a solution of 2,5-dichloro-*N*-(2-(*iso*propylthio)phenyl)pyrimidin-4-amine (20.0 g, 63.7 mol) in ethyl acetate (180 g), peracetic acid (33.4 g of a solution in acetic acid; 5.7 mmol/g) was added while maintaining an internal temperature of 20-30°C. After a reaction time of 16 hours, ethyl acetate (90 g) was added, followed by aqueous sodium sulfite (112 g of a 11% w/w solution) and maintaining an internal temperature of below 45°C. The phases were separated and to the organic phase was added Water (63 g), followed by aqueous NaOH (25% w/w solution) to adjust the pH 7-8. The phases were separated, the organic phase was dried over MgSO₄ and concentrated under vacuum. Ethanol (217 g) was added the mixture refluxed. After slow cooling to 0-5°C, the precipitate was filtrated and dried under vacuum to yield 2,5-dichloro-*N*-(2-(*iso*propylsulfonyl)phenyl)pyrimidin-4-amine (19.5 g, 88% yield) as a white powder. ¹H-NMR (400 MHz, CDCl₃): δ = 1.33 (d, *J* = 6.7 Hz, 6H), 3.22 (sept, *J* = 6.8 Hz, 1H), 7.31-7.35 (m, 1H), 7.72-7.76 (m, 1H), 7.92-7.94 (m, 1H), 8.31 (s, 1H), 8.63-8.65 (m, 1H), 10.07 (br s, 1H) ppm. ¹³C-NMR (100 MHz, CDCl₃): δ = 153, 56.1, 115.2, 122.7, 124.2, 124.5, 131.5, 135.20, 137.4, 155.6, 156.3, 157.8 ppm.

### Synthesis of 2,5-dichloro-N-(2-(isopropylsulfonyl)phenyl)pyrimidin-4-amine (C3, X=Cl), according to the following sequence:

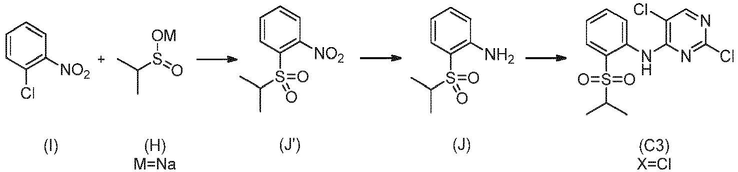

### 1-(Isopropylsulfonyl)-2-nitrobenzene (J')

Sodium propane-2-sulfinate (2.48 kg, 19.1 mol, 1.5 eq.) and 1-chloro-2-nitrobenzene (2.00 kg, 12.7 mol) were dissolved in DMSO (5.5 kg) and heated to 85 °C for 12 hours. After cooling to 20 °C, ice water (15 kg) was added followed by seeding with 1-(*iso*propylsulfonyl)-2-nitrobenzene (1 g). The reaction mixture was stirred for 30 min at 0-10°C, filtered and dried *under vacuum* to yield 1-(*iso*propylsulfonyl)-2-nitrobenzene (2.8 kg, 96% yield) as a grey solid.

### 2-(Isopropylsulfonyl)aniline (J)

A mixture of 1-(*iso*propylsulfonyl)-2-nitrobenzene (2.50 kg, 10.9 mol), ethanol (7.9 kg) and Pd/C (125 g, 5% w/w) in an autoclave was set under a hydrogen pressure of 3.4 bar and stirred for 48 hours at 40°C. After filtration of the catalyst, the filtrate was concentrated under vacuum to approximately 2.5 liters and the precipitate was filtered off. The filtrate was again concentrated under vacuum to approximately 0.8 liters and the precipitate was filtered off. The two filtered solids were combined and dried under vacuum to yield 2-(*Iso*propylsulfonyl)aniline (1.83 kg, 84% yield).

### 2,5-Dichloro-N-(2-(isopropylsulfonyl)phenyl)pyrimidin-4-amine (C3, X=Cl)

2,4,5-trichloropyrimidine (6.1 kg) was added onto 2-(*iso*propylsulfonyl)aniline (950 g, 4.77 mol) and DBU (181 g, 1.19 mol, 0.25 eq.), and the mixture was stirred for 7.5 hours at 80°C. After cooling to 25°C *n-*heptane (1.9 kg) was added and the mixture was stirred for 30 min. The mixture was cooled to -5°C, filtered and washed with *n*-heptane (325 g). The crude product was suspended in ethanol (4.5 kg), stirred for 12 hours at 25°C and then cooled to 0°C. After filtration the crude was dried under vacuum to yield 2,5-dichloro-*N*-(2-(*iso*propylsulfonyl)phenyl)pyrimidin-4-amine (1.07 kg, 65% yield). ¹H-NMR (400 MHz, CDCl₃): δ = 1.33 (d, *J* = 6.7 Hz, 6H), 3.22 (sept, *J* = 6.8 Hz, 1H), 7.31-7.35 (m, 1H), 7.72-7.76 (m, 1H), 7.92-7.94 (m, 1H), 8.31 (s, 1H), 8.63-8.65 (m, 1H), 10.07 (br. s, 1H) ppm. ¹³C-NMR (100 MHz, CDCl₃): δ = 153, 56.1, 115.2, 122.7, 124.2, 124.5, 131.5, 135.20, 137.4, 155.6, 156.3, 157.8 ppm.

### Synthesis of 5-chloro-N2-(2-isopropoxy-5-methyl-4-(piperidin-4-yl)phenyl)-N4-(2-(isopropylsulfonyl) phenyl)pyrimidine-2,4-diamine (ceritinib)

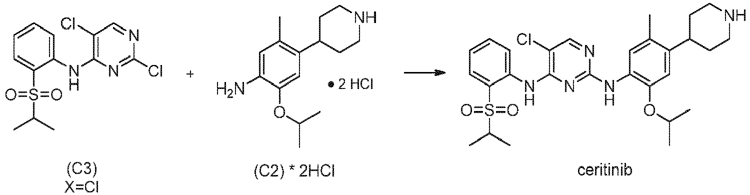

*Iso*propanol (445 kg) was added to 2,5-dichloro-*N*-(2-(*iso*propylsulfonyl)phenyl)pyrimidin-4-amine (70.2 kg, 203 mol, 1.15 eq.) and 2-lsopropoxy-5-methyl-4-(piperidin-4-yl)aniline dihydrochloride (56.7 kg, 176 mol). The mixture was heated for approx. 16 hours at reflux. Water (47 kg) was added and the mixture cooled to 0 °C. The solid was filtered and washed with *iso*propanol/water. To the wet product, isopropanol (680 kg) and water (55 kg) was added and the slurry heated to reflux. The obtained clear solution was cooled to 0 °C, filtered and dried under vacuum to yield 5-chloro-*N*2-(2-*iso*propoxy-5-methyl-4-(piperidin-4-yl)phenyl)-*N*4-(2-(*iso*propylsulfonyl)phenyl)pyrimidine-2,4-diamine dihydrochloride (= ceritinib dihydrochloride; 84.5 kg [t.q., contains 10% w/w isopropanol], 76.0 kg [100%], 68% yield).

Ethanol (155 kg) and water (113 kg) were added to 5-chloro-*N*2-(2-*iso*propoxy-5-methyl-4-(piperidin-4-yl)phenyl)-*N*4-(2-(*iso*propylsulfonyl)phenyl)pyrimidine-2,4- dihydrochloride; 45.0 kg [t.q., contains 10% w/w isopropanol], 40.5 kg [100%], 64.2 mol) and the mixture heated to 55 °C. Aqueous NaOH (147 L of a 1 M solution, 2.3 eq.) was added slowly and then cooled to 20 °C. After filtration, the product was recrystallized from ethanol and dried *under vacuum* to yield ceritinib (33.2 kg, 93% yield based on ceritinib dihydrochloride) as an almost white powder. ¹H-NMR (400 MHz, CDCl₃): δ = 1.33 (d, *J* = 6.8 Hz, 6H), 1.38 (d, *J* = 6.1 Hz, 6H), 1.59-1-78 (m, 5 H), 2.18 (s, 3H), 2.75-2.83 (m, 3H), 3.20-3.24 (m, 2H), overlaps 3.28 (sept, *J* = 6.8 Hz, 1H), 4.56 (sept, *J* = 6.1 Hz, 1H), 6.82 (s, 1H), 7.25-7.29 (m, 1 H), 7.56 (br. s, 1H), 7.64 (m, 1H), 7.94 (m, 1H), 8.01 (br. s, 1H), 8.16 (br. s, 1H), 8.60 (m, 1H), 9.51 (br. s, 1H) ppm. ¹³C-NMR (100 MHz, CDCl₃): δ = 15.4, 18.9, 22.3, 33.9, 38.6, 47.5, 55.4, 71.4, 105.7, 111.0, 120.6, 123.1, 123.7, 124.9, 126.7, 127.3, 131.2, 134.7, 138.3, 138.5, 144.7, 155.3, 155.4, 157.5 ppm, Elemental analysis: calculated (%) for C₂₈H₃₆ClN₅O₃S: C 60.26, H 6.50, N 12.55, O 8.60 Cl 6.35, S 5.74, found: C 60.15, H 6.45, N 12.72, O 8.58, Cl 6.43, S 5.67.)

## Claims

1. A process for preparing a compound of formula (C2-1), 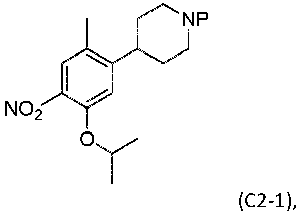 the process comprising the step of reacting a compound of formula (A) 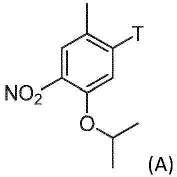 with a compound of formula (B) in a solvent 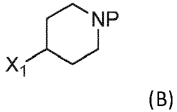 in the presence of at least one catalyst and a co-catalyst or additive, wherein
P is a protecting group;
T and X1 are independently selected from the group consisting of Cl, Br, I, CF₃SO₃, CH₃C₆H₄SO₃, O(t-BuC(O)), MgCl, MgBr, MgI, Sn(Alkyl)₃, Si(Alkyl)₃, Si(OAlkyl)₃, ZnCl, ZnBr, ZnI, ZnAlkyl, B(OH)₂, B(OC(CH₃)₂C(CH₃)₂O), 9-borabicyclo[3.3.1]nonane, bis(1,2-dimethylpropyl)borane, catecholborane, dicyclohexylboronyl, BF₃⁻, B-(N-methyliminodiacetic acid); the catalyst is Pd(PPh₃)₂Cl₂; and
the co-catalyst or additive is CuI.

2. The process for preparing a compound of formula (C2-1) according to claim 1, wherein T is Br and X₁ is ZnI.

3. A process for preparing ceritinib, or a salt thereof, 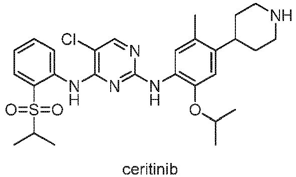 the process comprising the steps of:
i. preparing a compound of formula (C2-1) according to claim 1 or claim 2, 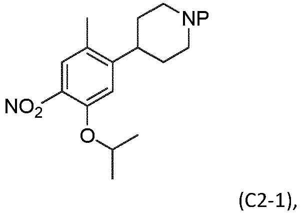
ii. preparing a compound of formula (C2) or a salt thereof from the compound of formula C(2-1), 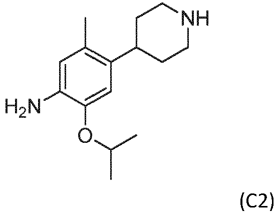
iii. providing a compound (C3), wherein X is selected from halogen (F, Cl, Br, I), alkoxy, aryloxy, alkylthio, sulfinyl, sulfonyl, and 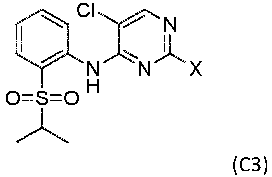
iv. reacting the compound of formula (C2), or a salt thereof, with the compound of formula (C3), to obtain ceritinib, or a salt thereof.

## Patentansprüche

1. Verfahren zur Herstellung einer Verbindung der Formel (C2-1), 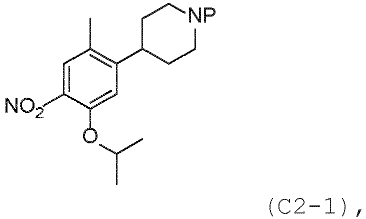 wobei das Verfahren den Schritt des Umsetzens einer Verbindung der Formel (A) 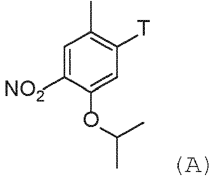 mit einer Verbindung der Formel (B) in einem Lösungsmittel 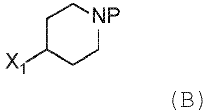 in Gegenwart mindestens eines Katalysators und eines Cokatalysators oder Additivs umfasst, wobei P für eine Schutzgruppe steht;
T und X1 unabhängig aus der Gruppe bestehend aus Cl, Br, I, CF₃SO₃, CH₃C₆H₄SO₃, O(t-BuC(O)), MgCl, MgBr, MgI, Sn(Alkyl)₃, Si(Alkyl)₃, Si(OAlkyl)₃, ZnCl, ZnBr, ZnI, ZnAlkyl, B(OH)₂, B(OC(CH₃)₂C(CH₃)₂O), 9-Borabicyclo[3.3.1]nonan, Bis(1,2-dimethylpropyl)boran, Catecholboran, Dicyclohexylboronyl, BF3⁻, B-(N-Methyliminodiessigsäure) ausgewählt sind;
es sich bei dem Katalysator um Pd(PPh₃)₂Cl₂ handelt und es sich bei dem Cokatalysator oder Additiv um CuI handelt.

2. Verfahren zur Herstellung einer Verbindung der Formel (C2-1) nach Anspruch 1, wobei T für Br steht und X1 für ZnI steht.

3. Verfahren zur Herstellung von Ceritinib oder einem Salz davon, 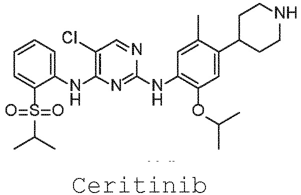 wobei das Verfahren die folgenden Schritte umfasst:
i. Herstellen einer Verbindung der Formel (C2-1) gemäß Anspruch 1 oder Anspruch 2, 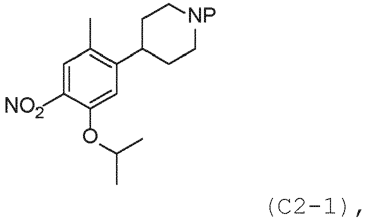
ii. Herstellen einer Verbindung der Formel (C2) oder eines Salzes davon aus der Verbindung der Formel (C2-1), 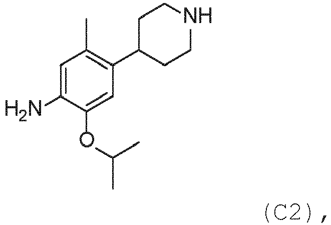
iii. Bereitstellen einer Verbindung (C3), wobei X aus Halogen (F, Cl, Br, I), Alkoxy, Aryloxy, Alkylthio, Sulfinyl und Sulfonyl ausgewählt ist, 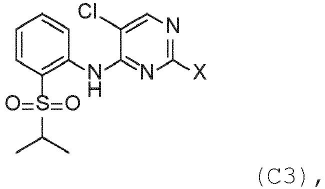
iv. Umsetzen der Verbindung der Formel (C2) oder eines Salzes davon mit der Verbindung der Formel (C3) zum Erhalt von Ceritinib oder einem Salz davon.

## Revendications

1. Procédé pour la préparation d'un composé de formule (C2-1), 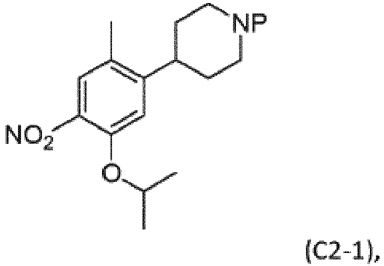 le procédé comprenant l'étape de mise en réaction d'un composé de formule (A) 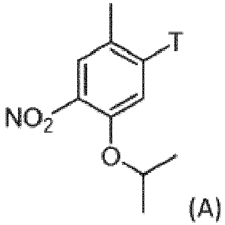 avec un composé de formule (B) dans un solvant 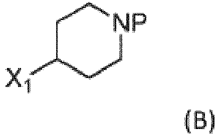 en présence d'au moins un catalyseur et d'un co-catalyseur ou d'un additif,
P étant un groupe protecteur ;
T et X1 étant indépendamment choisis dans le groupe constitué par Cl, Br, I, CF₃SO₃, CH₃C₆H₄SO₃, O(t-BuC(O)), MgCl, MgBr, MgI, Sn(alkyle)₃, Si(alkyle)₃, Si(Oalkyle)₃, ZnCl, ZnBr, ZnI, Znalkyle, B(OH)₂, B(OC(CH₃)₂C(CH₃)₂O), 9-borabicyclo[3.3.1]nonane, bis(1,2-diméthylpropyl)borane, catécholborane, dicyclohexylboronyle, BF3⁻, B-(N-acide méthyliminodiacétique) ;
le catalyseur étant Pd(PPh₃)₂Cl₂ ; et
le co-catalyseur ou l'additif étant CuI.

2. Procédé pour la préparation d'un composé de formule (C2-1) selon la revendication 1, T étant Br et X1 étant ZnI.

3. Procédé pour la préparation de céritinib, ou d'un sel correspondant, 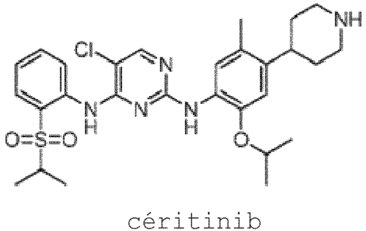 le procédé comprenant les étapes de :
i. préparation d'un composé de formule (C2-1) selon la revendication 1 ou la revendication 2, 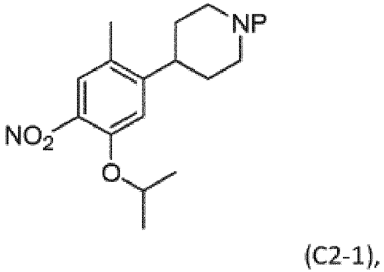
ii. préparation d'un composé de formule (C2) ou d'un sel correspondant à partir du composé de formule (C2-1), 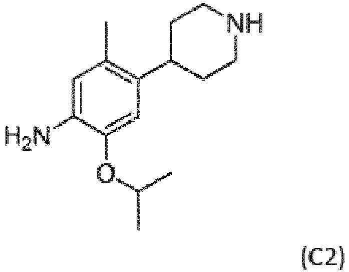
iii. mise à disposition d'un composé (C3), X étant choisi parmi halogène (F, Cl, Br, I), alcoxy, aryloxy, alkylthio, sulfinyle, sulfonyle, et 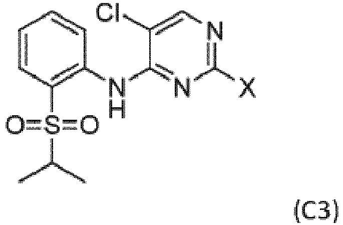
iv. mise en réaction du composé de formule (C2), ou d'un sel correspondant, avec le composé de formule (C3), pour obtenir le céritinib, ou un sel correspondant.
